**Europäisches Patentamt**

**European Patent Office**

**Office européen des brevets**

(19)

(11) Publication number : **0 452 695 B1**

## (12) EUROPEAN PATENT SPECIFICATION

(45) Date of publication of patent specification :
**03.08.94 Bulletin 94/31**

(51) Int. Cl.⁵ : **B01J 23/74,** B01J 23/78, C07C 37/56

(21) Application number : **91104471.7**

(22) Date of filing : **21.03.91**

(54) **Production of a catalyst and its use for producing phenol.**

(30) Priority : **17.04.90 JP 99333/90**
**30.07.90 JP 199161/90**
**11.12.90 JP 401303/90**
**27.12.90 JP 408265/90**
**27.12.90 JP 408266/90**
**27.12.90 JP 408267/90**
**27.12.90 JP 408268/90**

(43) Date of publication of application :
**23.10.91 Bulletin 91/43**

(45) Publication of the grant of the patent :
**03.08.94 Bulletin 94/31**

(84) Designated Contracting States :
**DE FR GB IT NL**

(56) References cited :
**FR-A- 2 280 428**
**US-A- 4 009 126**
**US-A- 4 215 018**
**US-A- 4 225 732**
**US-A- 4 567 157**

(73) Proprietor : **NKK CORPORATION**
**1-2 Marunouchi 1-chome,**
**Chiyoda-ku**
**Tokyo (JP)**

(72) Inventor : **Miki, Jun, c/o N.K.K. Corporation**
**1-2, Marunouchi 1-chome**
**Chiyoda-ku, Tokyo (JP)**
Inventor : **Suzuki, Toshifumi, c/o N.K.K.**
**Corporation**
**1-2, Marunouchi 1-chome**
**Chiyoda-ku, Tokyo (JP)**
Inventor : **Shikada, Tsutomu, c/o N.K.K.**
**Corporation**
**1-2, Marunouchi 1-chome**
**Chiyoda-ku, Tokyo (JP)**
Inventor : **Tate, Kazuhiko, c/o N.K.K.**
**Corporation**
**1-2, Marunouchi 1-chome**
**Chiyoda-ku, Tokyo (JP)**
Inventor : **Tachibana, Yakudo, c/o N.K.K.**
**Corporation**
**1-2, Marunouchi 1-chome**
**Chiyoda-ku, Tokyo (JP)**

(74) Representative : **Patentanwälte Grünecker,**
**Kinkeldey, Stockmair & Partner**
**Maximilianstrasse 58**
**D-80538 München (DE)**

EP 0 452 695 B1

Jouve, 18, rue Saint-Denis, 75001 PARIS

## Description

This invention relates to a process for producing a catalyst consisting essentially of iron oxide and nickel oxide, as well as to the use of such a catalyst for producing phenol from benzoic acid through vapor phase oxidation.

Heretofore, various catalysts and processes were developed for producing phenol from benzoic acid through catalytic oxidation in the vapor phase. For example, Japanese Patent KOKAI No. 57-11932 discloses a catalyst for producing phenol composed of at least one of copper compounds, vanadium compounds, silver compounds, lithium compounds, sodium compounds and magnesium compounds, and a process for producing phenol using the above catalyst. Another catalyst and process are disclosed in Japanese Patent KOKOKU No. 59-20384. The catalyst contains oxidized copper, zirconium and alkali metal, and the process uses them supported on an $\alpha$-alumina carrier as the catalyst. Moreover, Japanese Patent KOKOKU No. 64-934 discloses a process for producing phenol using an oxide catalyst composed of many kinds of metal elements, i.e. molybdenum as the essential element, at least one of vanadium, niobium and tantalum, at least one of copper, silver, manganese, iron, cobalt, nickel, rhodium, palladium and platinum, and at least one of thallium, alkali metals and alkaline earth metals.

However, the catalyst disclosed in Japanese Patent KOKAI No. 57-11932 is insufficient in the yield of phenol. Actually, the maximum conversion of benzoic acid was 50.5 %, and the maximum selectivity to phenol was 88.6 %, and therefore, the maximum yield was 44.7 %. Besides, when an exothermic reaction is conducted such as the oxidation reaction of benzoic aicd using the catalyst containing a copper compound, hot spots occur in the catalyst layer to progress calcining of the catalyst. As a result, the catalytic activity is degraded sharply. In the manufacturing process disclosed in Japanese Patent KOKOKU No. 59-20384, the yield of phenol is insufficient. That is, the maximum conversion of benzoic acid was 63.7 %, and the maximum selectivity to phenol was 82.2 %, and therefore, the maximum yield was 52.4 %. Moreover, byproducts such as diphenyl oxide are produced abundantly, and therefore, a purification process for the produced phenol is necessary. In the manufacturing process disclosed in Japanese Patent KOKOKU No. 64-934, the maximum conversion of benzoic acid was 75 %, and the maximum selectivity to phenol was 89 %. Therefore, the maximum yield was 66.8 %. Moreover, all of the above conventional processes are inferior in productivity due to low space time yield (the production amount of phenol per unit volume of catalyst per unit time), and therefore, they are disadvantageous in the industrial production of phenol.

A catalyst for reducing the amount of acetylenic compounds in a stream of organic components is known from US-A-4,009,126, said catalyst consisting essentially of a mixture of oxides, carbonates and hydroxides of Fe and Ni.

It is the object underlying the present invention to provide a novel process for producing a catalyst resistant to a high temperature and capable of producing phenol from benzoic acid through vapor phase oxidation in a high yield with less production of byproducts in a high space time yield.

According to the present invention this object is attained with a process for producing a catalyst consisting essentially of iron oxide and nickel oxide which comprises calcining a mixture of iron oxide or a precursor thereof with nickel oxide or a precursor thereof at a temperature of 600 to 900°C.

According to the present invention there is further povided the use of a catalyst prepared by the process of the present invention in a process for producing phenol from benzoic acid through vapor phase oxidation.

Preferred embodiments of the present invention are set forth in the dependent claims.

In the catalyst prepared with the present process a suitable ratio of nickel oxide/iron oxide is 0.1 to 10, preferably 0.3 to 5, more preferably 0.5 to 2 by weight. When the ratio of nickel oxide/iron oxide is greater than 10, the production of CO and $CO_2$ increases by the complete combustion resulting to reduce the selectivity to phenol. While, when the ratio is less than 0.1, the production of benzene increases resulting to reduce the selectivity to phenol. Most of the iron oxide may be $Fe_2O_3$, and most of the nickel oxide may be NiO, according to the preparation process of the catalyst.

The conversion of benzoic acid and the selectivity to phenol can be improved by adding to the catalyst an alkali metal compound, an alkaline earth metal compound or both of them.

The alkali metal compound is, for example, an oxide, a carbonate, a hydroxide, or a nitrate, and preferable compounds are oxides such as $Li_2O$, $Na_2O$, $K_2O$, $Rb_2O$ and $Cs_2O$, and $Na_2O$ and $K_2O$ are particularly preferred in view of the low production of CO and $CO_2$ and the high selectivity to phenol. The alkaline earth metal compound is e.g. also an oxide, a carbonate, a hydroxide or a nitrate and preferable compounds are oxides such as MgO, CaO, SrO and BaO; and CaO is particularly preferred.

The catalyst prepared according to the present process may be used on a support or carrier in a process for producing phenol from benzoic acid. A suitable metal oxide carrier is titania, magnesia, $\alpha$-alumina, silica gel, zirconia, tin oxide, lantharum oxide and the like, and titania, magnesia and $\alpha$-alumina are preferred in view of high conversion and high selectivity.

The catalyst can be supported on the metal oxide carrier by utilizing a conventional impregnation technique. The alkali metal compound and the alkaline earth metal compound may be added to the catalyst before, after or during supporting it on the metal oxide carrier.

The catalyst composition prepared by the impregnation is dried in the atmosphere at 90 to 150°C for 12 to 36 hours. The dried matter may be calcined in the atmosphere or a nitrogen gas atmosphere at 400 to 1,000°C for 1 to 10 hours. The conversion of benzoic acid and the selectivity to phenol can be improved by adding to the catalyst an alkali metal compound, an alkaline earth metal compound or both of them.

A suitable content of the alkali metal compound and that of the alkaline earth metal compound are 0.05 to 30 wt. %, preferably 0.1 to 10 wt. %, as the value converted to the oxide thereof, respectively. When the content is less than 0.05 wt. %, the production of CO and $CO_2$ increases resulting to reduce the selectivity to phenol. While, when the content is beyond 30 wt. %, the conversion of benzoic acid decreases. In the case of adding both of the alkali metal compound and the alkaline earth metal compound, each suitable content of both compounds converted to the oxide thereof is 0.01 to 20 wt. %, preferably 0.05 to 5 wt. %, respectively.

The nickel oxide-iron oxide catalyst may contain various other compounds. The process of the present invention may be carried out as follows.

The raw materials of iron oxide and nickel oxide may be, for example, nitrate, carbonate, organic acid salt, halide, hydroxide or oxide. The iron compound and the nickel compound are mixed by a conventional method such as, e.g., precipitation, kneading or impregnation.

Alternatively, iron oxide powder is mixed with nickel oxide powder, and the powder mixture may be pelletized by pressing. When the alkali metal compound or the alkaline earth metal compound is incorporated into the catalyst, for example, the alkali metal compound or the alkaline earth metal compound may be added to a gel mixture of iron hydroxide and nickel hydroxide as it is or in a state of a solution. Then, the mixture is dried and calcined. Besides, the alkali metal compound or the alkaline earth metal compound may be added to the mixture of iron oxide and nickel oxide, or may be added to the calcined mixture of iron oxide and nickel oxide by blending or impregnation. Furthermore, a mixture of iron oxide powder, nickel oxide powder and the alkali metal compound powder or the alkaline earth metal compound powder is prepared, and the powder mixture may be pelletized by pressing.

During preparing the iron oxide-nickel oxide catalysts of the invention, it is preferable to calcine iron oxide or a precursor thereof and nickel oxide or a precursor thereof, after they are mixed with each other. The calcination is conducted at 600 to 900°C, preferably 650 to 850 °C in the atmosphere or an inert gas atmosphere so as to crystallize at least one of iron oxide, nickel oxide and iron-nickel complex oxide. In general, when a catalyst prepared by a conventional method is calcined at a temperature higher than 600°C, the catalytic activity decreases due to the decrease of specific surface area. However, in the case of the present invention, the phenol-producing activity of the catalyst is improved to obtain a high conversion of benzoic acid and a high selectivity to phenol by the calcination at 600 to 900°C, although the specific surface area is decreased. When the calcination temperature is lower than 600°C, only CO and $CO_2$ production proceeds by the complete combustion. Phenol is produced in a small amount, and carbonaceous materials deposit on the surface of the catalyst. While, when the calcination temperature is higher than 900°C, the conversion of benzoic acid is sharply reduced, and the production of phenol is minor.

Phenol is produced from benzoic acid in the presence of at least one catalyst prepared with the present process and at that time, oxygen gas is supplied together with benzoic acid. The supply amount moles of oxygen gas is more than the theoretical amount against the supply amount moles of benzoic acid, and 0.5 to 50 times the amount of benzoic acid is preferred. When the supply amount of oxygen gas is more than 50 times that of benzoic acid, the complete combustion of benzoic acid frequently occurs. While, when the supply amount of oxygen gas is less than 0.5 time, a sufficient conversion of benzoic acid cannot occur. The oxygen gas may be in the form of air or pure oxygen gas, and may be diluted with an inert gas.

The reaction is, in general, conducted in the presence of water vapor, and the supply amount of water vapor is preferably 1 to 100 times that of benzoic acid in a molar ratio. A supply amount beyond 100 times is disadvantageous from an economical viewpoint. While, when the supply amount is less than about one time the amount of benzoic acid, the selectivity to phenol is degraded. The space velocity of the reaction gas, i.e. the sum of benzoic acid, oxygen-containing gas and water vapor, is preferably 100 to 50,000 $h^{-1}$, more preferably 2,000 to 20,000 $hr^{-1}$, furthermore preferably 5,000 to 20,000 $h^{-1}$. When the space velocity is less than 100 $h^{-1}$, the space time yield is insufficient. While, when the space velocity is beyond 50,000 $h^{-1}$, the conversion of benzoic acid is low. The reaction temperature is usually 200 to 600°C, and 350 to 500°C is preferred. When the reaction temperature is higher than 600°C, the selectivity to phenol decreases. While, when the reaction temperature is lower than 200 °C, the conversion of benzoic acid is insufficient. The reaction pressure may be any pressure capable of keeping the supplied raw materials in a vapor state, and it is usually ordinary pressure or a slightly pressurized condition.

The reaction apparatus may be of the fixed bed type or the fluidized bed type.

With the catalysts prepared according to the process of the invention a high conversion of benzoic acid and a high selectivity to phenol is observed; and phenol can be produced in a high yield, particularly in a high space time yield through the use of the catalyst in a process for producing phenol from benzoic acid by vapor phase oxidation.

EXAMPLES

Examples 1 and 2

3.9 g of nickel nitrate and 5.1 g of iron nitrate ($Fe(NO_3)_3 \cdot 9H_2O$) were dissolved in 30 ml of pure water, and 18 g of titanium dioxide were impregnated in the solution with stirring. After drying at 120°C, the dried matter was calcined in the atmosphere at 500°C for 3 h to obtain a catalyst having a ratio by weight of 5 : 5 : 90 as $NiO : Fe_2O_3 : TiO_2$.

Comparative Example 1

200 g of nickel nitrate were dissolved in 500 ml of pure water, and 100 g of sodium hydroxide were dissolved in 500 ml of pure water. Both solutions were added dropwise to $2\ell$ of pure water so as to maintain a pH of 7-8. After adding, the mixture was stirred for 1 h. The precipitates produced were filtered and washed. The cake was dried in the atmosphere at 120°C for 24 h, and then calcined in the atmosphere at 800°C for 4 h to obtain a catalyst.

Comparative Example 2

A catalyst was prepared according to Example 1 described in Japanese Patent KOKOKU No. 64-934.

Comparative Example 3

A catalyst was prepared according to Reference Example 1 described in Japanese Patent KOKOKU No. 59-20384.

Comparative Example 4

200 g of iron nitrate were dissolved in 500 ml of pure water, and 100 g of sodium hydroxide were dissolved in 500 ml of pure water. Both solutions were added dropwise to $2\ell$ of pure water so as to maintain a pH of 7-8. After adding, the mixture was stirred for 1 h. The precipitates produced were filtered and washed. The cake was dried in the atmosphere at 120°C for 24 h, and then calcined in the atmosphere at 800°C for 4 h to obtain a catalyst.

Production of Phenol

Each catalyst was crushed and sieved to a prescribed mesh size, and the amount described in Table 1 or 2 was placed in a quartz tube having an inner diameter of 20 mm. Benzoic acid, water vapor, air and nitrogen gas were supplied at the rate described in Table 1 or 2, and allowed to react at the temperature described in Table 1 or 2.

The experimental results are summarized in Table 1 and 2.

Table 1

| | Example 1 | Example 2 |
|---|---|---|
| Catalyst Composition (wt.%) | NiO-Fe$_2$O$_3$-TiO$_2$ (5:5:90) | NiO-Fe$_2$O$_3$-TiO$_2$ (5:5:90) |
| Catalyst Amount (g) | 20 | 5 |
| Reaction Temperature (°C) | 400 | 400 |
| Supply Rate of Benzoic Acid ($10^{-4}$ mol/min) | 1.45 | 2.70 |
| Supply Rate of Water Vapor ($10^{-3}$ mol/min) | 1.45 | 8.10 |
| Supply Rate of Air (ml/min) | 32 | 12 |
| Supply Rate of Nitrogen Gas (ml/min) | 49 | 60 |
| Conversion of Benzoic Acid (%) | 49 | 25 |
| Selectivity (%) | | |
| Phenol | 75 | 75 |
| Benzene | 23 | 11 |
| CO, CO$_2$ | 1 | 11 |
| Yield of Phenol (%) | 49x0.75 | 25x0.75 |

EP 0 452 695 B1

Table 2

| | Comparative 1 | Comparative 2 | Comparative 3 | Comparative 4 |
|---|---|---|---|---|
| Catalyst Composition (wt. ratio) | NiO (100) | $MoO_3-V_2O_5-CuO-Na_2O-Al_2O_3$ (3.9:3.7:3.8:5.9:82.7) | $CuO-ZnO-K_2O-Al_2O_3$ (4.0:3.0:3.6:89.4) | $Fe_2O_3$ (100) |
| Catalyst Amount (g) | 5 | 5 | 5 | 5 |
| Catalyst Calcining Temperature (°C) | 800 | 600 | 500 | 800 |
| Reaction Temperature ( C) | 400 | 300 | 300 | 400 |
| Supply Rate of Benzoic Acid ($10^{-4}$ mol/min) | 2.70 | 2.70 | 2.70 | 2.70 |
| Supply Rate of Water Vapor ($10^{-3}$ mol/min) | 8.10 | 8.10 | 8.10 | 8.10 |
| Supply Rate of Air (ml/min) | 12 | 12 | 12 | 12 |
| Supply Rate of Nitrogen Gas (ml/min) | 60 | 60 | 60 | 60 |
| Conversion of Benzoic Acid (%) | 8 | 48 | 25 | 0.1 |
| Selectivity (%) | | | | |
| Phenol | 15 | 80 | 70 | 10 |
| Benzene | 16 | 3 | 5 | 3 |
| $CO, CO_2$ | 68 | 16 | 19 | 85 |
| Yield of Phenol (%) | 8x0.15 | 48x0.80 | 25x0.70 | 0.1x0.10 |

EP 0 452 695 B1

Examples 3 and 4

200 g of iron nitrate (Fe(NO$_3$)$_3$·9H$_2$O) and 154 g of nickel nitrate (Ni(NO$_3$)$_2$·6H$_2$O) were dissolved in 500 ml of pure water, and 100 g of sodium hydroxide were dissolved in 500 ml of pure water. Both solutions were added dropwise to 2ℓ of pure water at ordinary temperature so as to maintain a pH of 7-8. After adding, the mixture was stirred for 1 h. The precipitates produced were filtered and washed. The cake was dried in the atmosphere at 120°C for 24 h, and then calcined in the atmosphere at 800°C for 4 h to obtain a catalyst having a ratio by weight of 50 : 50 as NiO : Fe$_2$O$_3$.

Example 5

Using 59 g of nickel nitrate and 328 g of iron nitrate, a catalyst was prepared with the same method as Examples 3 and 4. The catalyst obtained had a ratio by weight of 19 : 81 as NiO : Fe$_2$O$_3$.

Example 6

Using 108 g of nickel nitrate and 200 g of iron nitrate, a catalyst was prepared with the same method as Examples 3 and 4. The catalyst obtained had a ratio by weight of 44 : 56 as NiO : Fe$_2$O$_3$.

Example 7

Using 202 g of nickel nitate and 142 g of iron nitrate, a catalyst was prepared with the same method as Examples 3 and 4. The catalyst obtained had a ratio by weight of 65 : 35 as NiO : Fe$_2$O$_3$.

Example 8

Except that the calcining temperature was set at 700°C, a catalyst was prepared with the same method as Examples 3 and 4.

Example 9

Except that the calcining temperature was set at 600°C, a catalyst was prepared in the same method as Examples 3 and 4.

Comparative Example 5

Except that the calcining temperature was set at 500°C, a catalyst was prepared in the same method as Examples 3 and 4.

Comparative Example 6

Except that the calcining temperature was set at 400°C, a catalyst was prepared in the same method as Examples 3 and 4.

Comparative Example 7

Except that the calcining temperature was set at 1,000 °C, a catalyst was prepared in the same method as Examples 3 and 4.

Production of Phenol

Each catalyst was crushed and sieved to a prescribed mesh size, and the amount described in Table 3 was placed in a quartz tube having an inner diameter of 20 mm. Benzoic acid, water vapor, air and nitrogen gas were supplied at the rate described in Table 3, and allowed to react at the temperature described in Table 3.

The experimental results are summarized in Table 3.

Table 3-1

| | Example 3 | Example 4 | Example 5 | Example 6 | Eaxmple 7 |
|---|---|---|---|---|---|
| Catalyst Composition (wt. ratio) | $NiO-Fe_2O_3$ (50:50) | $NiO-Fe_2O_3$ (50:50) | $NiO-Fe_2O_3$ (19:81) | $NiO-Fe_2O_3$ (44:56) | $NiO-Fe_2O_3$ (65:35) |
| Catalyst Amount (g) | 20 | 5 | 20 | 20 | 20 |
| Catalyst Calcining Temperature (°C) | 800 | 800 | 800 | 800 | 800 |
| Reaction Temperature (°C) | 400 | 400 | 400 | 400 | 400 |
| Supply Rate of Benzoic Acid ($10^{-4}$mol/min) | 1.20 | 1.20 | 1.60 | 1.20 | 1.40 |
| Supply Rate of Water Vapor ($10^{-3}$mol/min) | 3.60 | 6.00 | 6.00 | 3.60 | 6.35 |
| Supply Rate of Air (ml/min) | 27 | 27 | 27 | 27 | 27 |
| Supply Rate of Nitrogen Gas (ml/min) | 40 | 40 | 40 | 40 | 40 |
| Conversion of Benzoic Acid (%) | 100 | 94 | 62 | 87 | 46 |
| Selectivity (%) | | | | | |
| Phenol | 93 | 86 | 25 | 78 | 73 |
| Benzene | 5 | 11 | 75 | 18 | 15 |
| CO, $CO_2$ | 1 | 2 | tr | 3 | 8 |
| Yield of Phenol (%) | 100x0.93 | 94x0.86 | 62x0.25 | 87x0.78 | 46x0.73 |

EP 0 452 695 B1

Table 3-2

| | Example 8 | Example 9 | Comparative Example 5 | Comparative Example 6 | Comparative Example 7 |
|---|---|---|---|---|---|
| Catalyst Composition (wt. ratio) | $NiO-Fe_2O_3$ (50:50) | $NiO-Fe_2O_3$ (50:50) | $NiO-Fe_2O_3$ (50:50) | $NiO-Fe_2O_3$ (50:50) | $NiO-Fe_2O_3$ (19:81) |
| Catalyst Amount (g) | 5 | 5 | 5 | 5 | 5 |
| Catalyst Calcining Temperature (°C) | 700 | 600 | 500 | 400 | 1000 |
| Reaction Temperature (°C) | 400 | 400 | 400 | 400 | 400 |
| Supply Rate of Benzoic Acid ($10^{-4}$ mol/min) | 1.20 | 1.20 | 1.20 | 1.20 | 1.20 |
| Supply Rate of Water Vapor ($10^{-3}$ mol/min) | 6.00 | 6.00 | 6.00 | 6.00 | 6.00 |
| Supply Rate of Air (ml/min) | 27 | 27 | 27 | 27 | 27 |
| Supply Rate of Nitrogen Gas (ml/min) | 40 | 40 | 40 | 40 | 40 |
| Conversion of Benzoic Acid (%) | 89 | 94 | 32 | 30 | 0.2 |
| Selectivity (%) | | | | | |
| Phenol | 72 | 51 | tr | tr | tr |
| Benzene | 22 | 44 | tr | tr | tr |
| $CO$, $CO_2$ | 6 | 3 | 100 | 100 | tr |
| Yield of Phenol (%) | 89x0.72 | 94x0.51 | 32xtr | 30xtr | 0.2xtr |

EP 0 452 695 B1

Example 10

200 g of iron nitrate (Fe(NO$_3$)$_3$·9H$_2$O) and 144 g of nickel nitrate (Ni(NO$_3$)$_2$·6H$_2$O) were dissolved in 500 ml of pure water, and 100 g of sodium hydroxide were dissolved in 500 ml of pure water. Both solutions were added dropwise to 2 $\ell$ of pure water at ordinary temperature so as to maintain a pH of 7-8. After adding, the mixture was stirred for 1 h. The precipitates produced were filtered and washed. Subsequently, 100 ml of aqueous solution containing 1.62 g of calcium nitrate (Ca(NO$_3$)$_2$·4H$_2$O) were added to the gelatinous material, and stirred for 1h. The gelatinous material was dried in the atmosphere at 120°C for 24 h, and then calcined in the atmosphere at 800°C for 4 h to obtain a catalyst having a ratio by weight of 51.4 : 48.1 : 0.5 as Fe$_2$O$_3$ : NiO : CaO.

Example 11

Using 3.26 g of calcium nitrate, a catalyst was prepared with the same method as Example 10. The catalyst obtained had a ratio by weight of 51.2 : 47.8 : 1.0 as Fe$_2$O$_3$ : NiO : CaO.

Examples 12 and 13

200 g of iron nitrate and 144 g of nickel nitrate were dissolved in 500 ml of pure water, and 100 g of sodium hydroxide were dissolved in 500 ml of pure water. Both solutions were added dropwise to 2 $\ell$ of pure water at ordinary temperature so as to maintain a pH of 7-8. After adding, the mixture was stirred for 1 h. The precipitates produced were filtered and washed. The cake was dried in the atmosphere at 120°C for 24 h, and then calcined in the atmosphere at 800°C for 4 h. Subsequently, 49.5 g of the calcined matter were put in 200 ml of aqueous solution containing 2.11 g of calcium nitrate, and evaporated to dryness. The dried matter was further dried at 120°C for 24 h, and calcined again in the atmosphere at 500°C for 3 h to obtain a catalyst having a ratio by weight of 51.2 : 47.8 : 1.0 as Fe$_2$O$_3$: NiO: CaO.

Comparative Examples 8 to 12

The catalysts prepared in Comparatives Examples 1-4 were used, and the conditions for producing phenol were changed.

Production of Phenol

Each catalyst was crushed and sieved to a prescribed mesh size, and the amount described in Table 4 or 5 was placed in a quartz tube having an inner diameter of 20 mm. Benzoic acid, water vapor, air and nitrogen gas were supplied at the rate described in Table 4 or 5, and allowed to react at the temperature described in Table 4 or 5.

The experimental results are summarized in Table 4 and 5.

Table 4

| | Example 10 | Example 11 | Example 12 | Example 13 |
|---|---|---|---|---|
| Catalyst | | | | |
| Composition | $Fe_2O_3$-NiO-CaO | $Fe_2O_3$-NiO-CaO | $Fe_2O_3$-NiO-CaO | $Fe_2O_3$-NiO-CaO |
| (wt. ratio) | (51.4:48.1:0.5) | (51.2:47.8:1.0) | (51.2:47.8:1.0) | (51.2:47.8:1.0) |
| Calcining Temperature (°C) | 800 | 800 | 800 | 800 |
| Used Amount (ml) | 3.85 | 3.85 | 3.85 | 3.85 |
| Reacting Conditions | | | | |
| Reaction Temperature (°C) | 400 | 400 | 400 | 400 |
| Supply Gas Concentration (%) | | | | |
| Benzoic Acid | 7.4 | 6.7 | 7.9 | 10.1 |
| Water Vapor | 66.7 | 67.1 | 70.9 | 70.7 |
| Oxygen Gas | 5.2 | 5.2 | 4.3 | 3.8 |
| Nitrogen Gas | 20.7 | 20.9 | 17.0 | 15.4 |
| Space Velocity ($h^{-1}$) | 7730 | 7760 | 9180 | 10190 |
| Reaction Results | | | | |
| Conversion of Benzoic Acid (%) | 44.2 | 40.0 | 15.4 | 14.6 |
| Selectivity (%) | | | | |
| Phenol | 92.4 | 94.6 | 98.7 | 95.4 |
| Benzene | 7.3 | 4.1 | 1.1 | 3.7 |
| $CO, CO_2$ | 0.3 | 0.3 | tr | tr |
| Yield of Phenol (%) | 44.2x0.924 | 40.0x0.946 | 15.4x0.987 | 14.6x0.954 |
| Space Time Yield of Phenol (g/$\ell$·h) | 980 | 826 | 461 | 601 |

EP 0 452 695 B1

Table 5

| Catalyst | Comparative 8 | Comparative 9 | Comparative 10 | Comparative 11 | Comparative 12 |
|---|---|---|---|---|---|
| Composition (wt. ratio) | NiO (100) | $Fe_2O_3$ (100) | $MoO_3$-$V_2O_5$-CuO-$Na_2O$-$Al_2O_3$ (3.9:3.7:3.8:5.9:82.7) | $MoO_2$-$V_2O_5$-CuO-$Na_2O$-$Al_2O_3$ (3.9:3.7:3.8:5.9:82.7) | CuO-ZnO-$K_2O$-$Al_2O_3$ (4.0:3.0:3.6:89.4) |
| Calcining Temperature (°C) | 800 | 800 | 600 | 800 | 500 |
| Used Amount (ml) | 1.9 | 1.9 | 5.9 | 3.85 | 5.3 |
| Reacting Conditions | | | | | |
| Reaction Temperature (°C) | 400 | 400 | 300 | 300 | 300 |
| Supply Gas Concentration (%) | | | | | |
| Benzoic Acid | 2.3 | 2.3 | 2.3 | 4.0 | 2.3 |
| Water Vapor | 69.9 | 69.9 | 69.9 | 71.0 | 69.9 |
| Oxygen Gas | 4.7 | 4.7 | 4.7 | 5.1 | 4.7 |
| Nitrogen Gas | 23.1 | 23.1 | 23.1 | 20.0 | 23.1 |
| Space Velocity ($h^{-1}$) | 8200 | 8200 | 2640 | 9380 | 2960 |
| Reaction Results | | | | | |
| Conversion of Benzoic Acid (%) | 8.0 | 0.1 | 47.8 | 16.3 | 25.1 |
| Selectivity (%) | | | | | |
| Phenol | 15.1 | 10.4 | 79.7 | 41.3 | 70.4 |
| Benzene | 15.9 | 3.1 | 2.9 | 14.2 | 4.6 |
| $CO$, $CO_2$ | 69.0 | 85.3 | 16.3 | 30.5 | 18.7 |
| Yield of Phenol (%) | 8.0x0.151 | 0.1x0.104 | 47.8x0.797 | 16.3x0.413 | 25.1x0.704 |
| Space Time Yield of Phenol ($g/\ell \cdot h$) | 10 | 0.1 | 99 | 96 | 50 |

EP 0 452 695 B1

Example 14

200 g of iron nitrate (Fe(NO$_3$)$_3$·9H$_2$O) and 144 g of nickel nitrate (Ni(NO$_3$)·6H$_2$O) were dissolved in 500 ml of pure water, and 100 g of sodium hydroxide were dissolved in 500 ml of pure water. Both solutions were added dropwise to 2$\ell$ of pure water at ordinary temperature so as to maintain a pH of 7-8. After adding, the mixture was stirred for 1 h. The precipitates produced were filtered and washed. The cake was dried in the atmosphere at 120°C for 24 h, and then calcined in the atmosphere at 800°C for 4 h. Subsequently, 49.75 g of the calcined matter were put in 200 ml of aqueous solution containing 0.30 g of potassium hydroxide (KOH), and evaporated to dryness. The dried matter was dried in the atmosphere at 120°C for 24 h, and calcined again in the atmosphere at 800°C for 4 h to obtain a catalyst having a ratio by weight of 51.4 : 48.1 : 0.5 as Fe$_2$O$_3$ : NiO : K$_2$O.

Examples 15 to 18

Using 49.5 g of the calcined matter and 0.6 g of potassium hydroxide, a catalyst was prepared with the same method as in Example 14. The catalyst obtained had a ratio by weight of 51.2 : 47.8 : 1.0 as Fe$_2$O$_3$ : NiO K$_2$O.

Example 19

200 g of iron nitrate (Fe(NO$_3$)$_3$·9H$_2$O) and 144 g of nickel nitrate (Ni(NO$_3$)$_2$·6H$_2$O) were dissolved in 500 ml of pure water, and 100 g of sodium hydroxide were dissolved in 500 ml of pure water. Both solutions were added dropwise to 2$\ell$ of pure water at ordinary temperature so as to maintain a pH of 7-8. After adding the mixture was stirred for 1 h. The precipitates produced were filtered and washed. Subsequently, 100 ml of aqueous solution containing 1.77 g of sodium carbonate (Na$_2$CO$_3$·10H$_2$O) were added to the gelatinous material, and stirred for 1 h. The gelatinous material was dried in the atmosphere at 120°C for 24 h, and then calcined in the atmosphere at 800°C for 4 h to obtain a catalyst having a ratio by weight of 51.4 : 48.1 : 0.5 as Fe$_2$O$_3$ : NiO : Na$_2$O.

Examples 20 to 23

Except of changing the amount of sodium carbonate, various catalysts were prepared with the same method as in Example 27. The compositions of the obtained catalysts were 51.7 : 48.2 : 0.05, 51.2 : 47.8 : 1.0, 50.6 : 47.4 : 2.0 and 49.1 : 45.9 : 5.0 as the ratio by weight of Fe$_2$O$_3$ : NiO : Na$_2$O, respectively.

Examples 24 to 26

Except of changing the calcining temperature, various catalysts were prepared with the same method as in Example 19.

Examples 27 to 30

Except of changing the amount of ferric nitrate and nickel nitrate, various catalysts were prepared with the same method as in Example 19. The compositions of the obtained catalysts were 21.0 : 78.5 : 0.5, 34.6 : 64.9 : 0.5, 62.8 : 36.7 : 0.5 and 67.8 : 31.7 : 0.5 as the ratio by weight of Fe$_2$O$_3$ : NiO : Na$_2$O, respectively.

Examples 31 to 35

200 g of iron nitrate (Fe(NO$_3$)$_3$·9H$_2$O) and 144 g of nickel nitrate (Ni(NO$_3$)$_2$·6H$_2$O) were dissolved in 500 ml of pure water, and 100 g of sodium hydroxide were dissolved in 500 ml of pure water. Both solutions were added dropwise to 2$\ell$ of pure water at ordinary temperature so as to maintain a pH of 7-8. After adding, the mixture was stirred for 1 h. The precipitates produced were filtered and washed. Subsequently, 100 ml of aqueous solution containing 1.77 g of sodium carbonate (Na$_2$CO$_3$·10H$_2$O) were added to the gelatinous material, and stirred for 1 h. The gelatinous material was dried in the atmosphere at 120°C for 24 h, then calcined in the atmosphere at 800°C for 4 h to obtain a catalyst having a ratio by weight of 51.4 : 48.1 : 0.5 as Fe$_2$O$_3$ : N$_2$O : Na$_2$O.

Example 36

Using 3.75 g of sodium carbonate, a catalyst was prepared with the same method as in Examples 31 to 35. The catalyst obtained had a ratio by weight of 51.2 : 47.8 : 1.0 as $Fe_2O_3$ : NiO : $Na_2O$.

Example 37

200 g of iron nitrate ($Fe(NO_3)_3 \cdot 9H_2O$) and 144 g of nickel nitrate ($Ni(NO_3)_2 \cdot 6H_2O$) were dissolved in 500 ml of pure water, and 100 g of sodium hydroxide were dissolved in 500 ml of pure water. Both solutions were added dropwise to $2\ell$ of pure water at ordinary temperature so as to maintain a pH of 7-8. After adding, the mixture was stirred for 1 h. The precipitates produced were filtered and washed. The cake was dried in the atmosphere at 120°C for 24 h, and then calcined in the atmosphere at 800°C for 4 h. Subsequently, 49.5 g of the calcined matter were impregnated in 200 ml of aqueous solution containing 0.65 g of sodium hydroxide (NaOH), and evaporated to dryness. The dried matter was dried in the atmosphere at 120°C for 24 h, and calcined again in the atmosphere at 500°C for 3 h to obtain a catalyst having a ratio by weight of 51.2 : 47.8 : 1.0 as $Fe_2O_3$ : NiO : $Na_2O$.

Example 38

Using 2.22 g of sodium carbonate, a catalyst was prepared with the same method as in Examples 31 to 35. The catalyst obtained had a ratio by weight of 51.2 : 47.8 : 1.0 as $Fe_2O_3$ : NiO : $Na_2O$.

Comparative Examples 13 and 14

Changing the calcining temperature to 500°C or 1,000°C, catalysts were prepared with the same method as in Example 19.

Production of Phenol

Each catalyst was crushed and sieved to a prescribed mesh size, and the amount described in Table 6 or 7 was placed in a quartz tube having an inner diameter of 20 mm. Benzoic acid, water vapor, air and nitrogen gas were supplied at the rate described in Table 6 or 7, and allowed to react at the temperature described in Table 6 or 7.

The experimental results are summarized in Table 6 and 7. The experimental results of Comparative Examples 8 to 12 are also shown in Table 7.

Table 6-1

| | Example 14 | Example 15 | Example 16 | Example 17 | Example 18 |
|---|---|---|---|---|---|
| Catalyst | | | | | |
| Composition (wt. ratio) | $Fe_2O_3$-NiO-$K_2O$ (51.4:48.1:0.5) | $Fe_2O_3$-NiO-$K_2O$ (51.2:47.8:1.0) | $Fe_2O_3$-NiO-$K_2O$ (51.2:47.8:1.0) | $Fe_2O_3$-NiO-$K_2O$ (51.2:47.8:1.0) | $Fe_2O_3$-NiO-$K_2O$ (51.2:47.8:1.0) |
| Calcining Temperature (°C) | 800 | 800 | 800 | 800 | 800 |
| Used Amount (ml) | 3.85 | 3.85 | 3.85 | 3.85 | 3.85 |
| Reacting Conditions | | | | | |
| Reaction Temperature (°C) | 400 | 400 | 400 | 400 | 400 |
| Supply Gas Concentration (%) | | | | | |
| Benzoic Acid | 6.7 | 7.5 | 3.9 | 7.0 | 3.4 |
| Water Vapor | 66.7 | 75.2 | 70.6 | 63.4 | 83.9 |
| Oxygen Gas | 5.3 | 3.5 | 5.1 | 5.9 | 2.6 |
| Nitrogen Gas | 21.3 | 13.8 | 20.4 | 23.7 | 10.2 |
| Space Velocity ($h^{-1}$) | 7590 | 11360 | 9180 | 5710 | 3420 |
| Reaction Results | | | | | |
| Conversion of Benzoic Acid (%) | 67.9 | 45.6 | 40.4 | 37.2 | 37.0 |
| Selectivity (%) | | | | | |
| Phenol | 91.1 | 83.4 | 90.1 | 93.5 | 95.3 |
| Benzene | 8.6 | 13.3 | 5.4 | 6.1 | 3.3 |
| $CO, CO_2$ | 0.3 | 0.6 | 1.3 | 0.2 | 1.3 |
| Yield of Phenol (%) | 67.9x0.911 | 45.6x0.834 | 40.4x0.901 | 37.2x0.935 | 37.0x0.953 |
| Space Time Yield of Phenol (g/$\ell$·h) | 1320 | 1370 | 547 | 588 | 169 |

EP 0 452 695 B1

Table 6-2

| | Example 19 | Example 20 | Example 21 | Example 22 | Example 23 |
|---|---|---|---|---|---|
| Catalyst | | | | | |
| Composition (wt. ratio) | $Fe_2O_3$-NiO-$Na_2O$ (51.4:48.1:0.5) | $Fe_2O_3$-NiO-$Na_2O$ (51.7:48.2:0.05) | $Fe_2O_3$-NiO-$Na_2O$ (51.2:47.8:1.0) | $Fe_2O_3$-NiO-$Na_2O$ (50.6:47.4:2.0) | $Fe_2O_3$-NiO-$Na_2O$ (49.1:45.9:5.0) |
| Calcining Temperature (°C) | 800 | 800 | 800 | 800 | 800 |
| Used Amount (ml) | 3.85 | 3.85 | 3.85 | 3.85 | 3.85 |
| Reacting Conditions | | | | | |
| Reaction Temperature (°C) | 400 | 400 | 400 | 400 | 400 |
| Supply Gas Concentration (%) | | | | | |
| Benzoic Acid | 6.8 | 4.8 | 6.7 | 6.7 | 6.7 |
| Water Vapor | 67.6 | 81.0 | 67.1 | 67.1 | 67.1 |
| Oxygen Gas | 5.1 | 2.9 | 5.2 | 5.2 | 5.2 |
| Nitrogen Gas | 20.5 | 11.4 | 20.9 | 20.9 | 20.9 |
| Space Velocity ($h^{-1}$) | 7700 | 9660 | 7580 | 7610 | 7600 |
| Reaction Results | | | | | |
| Conversion of Benzoic Acid (%) | 95.2 | 31.7 | 72.0 | 71.6 | 73.4 |
| Selectivity (%) | | | | | |
| Phenol | 90.1 | 83.3 | 86.0 | 87.1 | 49.2 |
| Benzene | 7.6 | 15.2 | 11.5 | 12.9 | 46.3 |
| $CO$, $CO_2$ | 1.9 | 1.5 | 0.7 | 0.1 | 1.3 |
| Yield of Phenol (%) | 95.2x0.901 | 31.7x0.833 | 72.0x0.860 | 71.6x0.871 | 73.4x0.492 |
| Space Time Yield of Phenol ($g/\ell \cdot h$) | 1885 | 514 | 1320 | 1334 | 772 |

EP 0 452 695 B1

Table 6-3

| | Example 24 | Example 25 | Example 26 | Example 27 | Example 28 |
|---|---|---|---|---|---|
| **Catalyst** | | | | | |
| Composition (wt. ratio) | $Fe_2O_3$–NiO–$Na_2O$ (51.4:48.1:0.5) | $Fe_2O_3$–NiO–$Na_2O$ (51.4:48.1:0.5) | $Fe_2O_3$–NiO–$Na_2O$ (51.4:48.1:0.5) | $Fe_2O_3$–NiO–$Na_2O$ (21.0:78.5:0.5) | $Fe_2O_3$–NiO–$Na_2O$ (34.6:64.9:0.5) |
| Calcining Temperature (°C) | 700 | 600 | 900 | 800 | 800 |
| Used Amount (ml) | 3.85 | 3.85 | 3.85 | 3.85 | 3.85 |
| **Reacting Conditions** | | | | | |
| Reaction Temperature (°C) | 400 | 400 | 400 | 400 | 400 |
| Supply Gas Concentration (%) | | | | | |
| Benzoic Acid | 6.8 | 6.7 | 6.4 | 6.7 | 6.7 |
| Water Vapor | 67.6 | 67.1 | 67.7 | 67.2 | 67.1 |
| Oxygen Gas | 5.1 | 5.2 | 5.2 | 5.2 | 5.2 |
| Nitrogen Gas | 20.5 | 20.9 | 20.7 | 20.9 | 20.9 |
| Space Velocity ($h^{-1}$) | 7920 | 7760 | 7340 | 7930 | 7330 |
| **Reaction Results** | | | | | |
| Conversion of Benzoic Acid (%) | 91.3 | 83.7 | 50.3 | 79.5 | 91.2 |
| Selectivity (%) | | | | | |
| Phenol | 79.6 | 75.1 | 60.7 | 76.3 | 90.5 |
| Benzene | 17.9 | 21.6 | 23.5 | 14.9 | 7.3 |
| $CO$, $CO_2$ | 2.5 | 3.3 | 15.8 | 8.8 | 2.2 |
| Yield of Phenol (%) | 91.3x0.796 | 83.7x0.751 | 50.3x0.607 | 79.5x0.763 | 91.2x0.905 |
| Space Time Yield of Phenol ($g/\ell \cdot h$) | 1642 | 1370 | 602 | 1352 | 1701 |

EP 0 452 695 B1

Table 6–4

| | Example 29 | Example 30 | Example 31 | Example 32 | Example 33 |
|---|---|---|---|---|---|
| Catalyst | | | | | |
| Composition (wt. ratio) | $Fe_2O_3$–NiO–$Na_2$O (62.8:36.7:0.5) | $Fe_2O_3$–NiO–$Na_2$O (67.8:31.7:0.5) | $Fe_2O_3$–NiO–$Na_2$O (51.4:48.1:0.5) | $Fe_2O_3$–NiO–$Na_2$O (51.4:48.1:0.5) | $Fe_2O_3$–NiO–$Na_2$O (51.4:48.1:0.5) |
| Calcining Temperature (°C) | 800 | 800 | 800 | 800 | 800 |
| Used Amount (ml) | 3.85 | 3.85 | 3.85 | 3.85 | 3.85 |
| Reacting Conditions | | | | | |
| Reaction Temperature (°C) | 400 | 400 | 350 | 370 | 400 |
| Supply Gas Concentration (%) | | | | | |
| Benzoic Acid | 6.8 | 6.6 | 7.6 | 7.7 | 7.8 |
| Water Vapor | 67.1 | 67.3 | 70.4 | 69.2 | 69.8 |
| Oxygen Gas | 5.2 | 5.2 | 4.4 | 4.6 | 4.5 |
| Nitrogen Gas | 20.9 | 20.9 | 17.6 | 18.5 | 18.0 |
| Space Velocity ($h^{-1}$) | 7780 | 7760 | 8590 | 8440 | 8700 |
| Reaction Results | | | | | |
| Conversion of Benzoic Acid (%) | 88.6 | 65.8 | 46.0 | 67.7 | 83.8 |
| Selectivity (%) | | | | | |
| Phenol | 68.1 | 43.5 | 87.6 | 84.3 | 89.5 |
| Benzene | 28.6 | 54.4 | 12.5 | 13.1 | 9.0 |
| CO, $CO_2$ | 3.3 | 2.1 | tr | tr | 0.4 |
| Yield of Phenol (%) | 88.6x0.681 | 65.8x0.435 | 46.0x0.876 | 67.7x0.843 | 83.8x0.895 |
| Space Time Yield of Phenol (g/ℓ·h) | 1339 | 615 | 1104 | 1556 | 2121 |

EP 0 452 695 B1

Table 6–5

| | Example 34 | Example 35 | Example 36 | Example 37 | Example 38 |
|---|---|---|---|---|---|
| **Catalyst** | | | | | |
| Composition (wt. ratio) | $Fe_2O_3$–NiO–$Na_2O$ (51.4:48.1:0.5) | $Fe_2O_3$–NiO–$Na_2O$ (51.4:48.1:0.5) | $Fe_2O_3$–NiO–$Na_2O$ (51.2:47.8:1.0) | $Fe_2O_3$–NiO–$Na_2O$ (51.2:47.8:1.0) | $Fe_2O_3$–NiO–$Na_2O$ (51.2:47.8:1.0) |
| Calcining Temperature (°C) | 800 | 800 | 800 | 800 | 800 |
| Used Amount (ml) | 3.85 | 3.85 | 3.85 | 3.85 | 3.85 |
| **Reacting Conditions** | | | | | |
| Reaction Temperature (°C) | 425 | 450 | 400 | 400 | 400 |
| Supply Gas Concentration (%) | | | | | |
| Benzoic Acid | 7.7 | 7.7 | 5.2 | 4.0 | 5.9 |
| Water Vapor | 69.2 | 69.2 | 72.5 | 71.1 | 70.6 |
| Oxygen Gas | 4.6 | 4.6 | 4.6 | 5.0 | 4.7 |
| Nitrogen Gas | 18.5 | 18.5 | 17.8 | 19.9 | 18.8 |
| Space Velocity ($h^{-1}$) | 8830 | 8730 | 8770 | 9360 | 8300 |
| **Reaction Results** | | | | | |
| Conversion of Benzoic Acid (%) | 76.9 | 89.4 | 76.9 | 100 | 53.9 |
| Selectivity (%) | | | | | |
| Phenol | 81.8 | 69.3 | 87.5 | 91.1 | 81.7 |
| Benzene | 17.1 | 23.6 | 10.7 | 7.8 | 15.0 |
| CO, $CO_2$ | 0.9 | 3.8 | 0.4 | 0.6 | 2.0 |
| Yield of Phenol (%) | 76.9x0.818 | 89.4x0.693 | 76.9x0.875 | 100x0.911 | 53.9x0.817 |
| Space Time Yield of Phenol (g/ℓ·h) | 1797 | 1675 | 1288 | 1430 | 905 |

EP 0 452 695 B1

Table 7

| | Comparative 8 | Comparative 9 | Comparative 10 | Comparative 11 | Comparative 12 | Comparative 13 | Comparative 14 |
|---|---|---|---|---|---|---|---|
| **Catalyst** | | | | | | | |
| Composition | NiO | $Fe_2O_3$ | $MoO_3$-$V_2O_5$-CuO-$Na_2O$-$Al_2O_3$ | $MoO_3$-$V_2O_5$-CuO-$Na_2O$-$Al_2O_3$ | CuO-ZnO-$K_2O$-$Al_2O_3$ | $Fe_2O_3$-NiO-$Na_2O$ | $Fe_2O_3$-NiO-$NaO_2$ |
| (wt. ratio) | (100) | (100) | (3.9:3.7:3.8: 5.9:82.7) | (3.9:3.7:3.8: 5.9:82.7) | (4.0:3.0: 3.6:89.4) | (51.4:48.1:0.5) | (51.4:48.1:0.5) |
| Calcining Temperature (°C) | 800 | 800 | 600 | 600 | 500 | 500 | 1000 |
| Used Amount (ml) | 1.9 | 1.9 | 5.9 | 3.85 | 5.3 | 3.85 | 3.85 |
| **Reacting Conditions** | | | | | | | |
| Reaction Temperature (°C) | 400 | 400 | 300 | 300 | 300 | 400 | 400 |
| Supply Gas Concentration (%) | | | | | | | |
| Benzoic Acid | 2.3 | 2.3 | 2.3 | 4.0 | 2.3 | 6.7 | 6.7 |
| Water Vapor | 69.9 | 69.9 | 69.9 | 71.0 | 69.9 | 67.1 | 67.1 |
| Oxygen Gas | 4.7 | 4.7 | 4.7 | 5.1 | 4.7 | 5.2 | 5.2 |
| Nitrogen Gas | 23.1 | 23.1 | 23.1 | 20.0 | 23.1 | 20.9 | 20.9 |
| Space Velocity $(h^{-1})$ | 8200 | 8200 | 2640 | 9380 | 2960 | 7780 | 7630 |
| **Reaction Results** | | | | | | | |
| Conversion of Benzoic Acid (%) | 8.0 | 0.1 | 47.8 | 16.3 | 25.1 | 11.8 | 0.1 |
| Selectivity (%) | | | | | | | |
| Phenol | 15.1 | 10.4 | 79.7 | 41.3 | 70.4 | 1.1 | tr |
| Benzene | 15.9 | 3.1 | 2.9 | 14.2 | 4.6 | 7.6 | tr |
| $CO$, $CO_2$ | 69.0 | 85.3 | 16.3 | 30.5 | 18.7 | 91.3 | tr |
| Yield of Phenol (%) | 8.0x0.151 | 0.1x0.104 | 47.8x0.797 | 16.3x0.413 | 25.1x0.704 | 11.8x0.11 | tr |
| Space Time Yield of Phenol $(g/\ell \cdot h)$ | 10 | 0.1 | 99 | 96 | 50 | 3 | tr |

EP 0 452 695 B1

Example 39 to 41

200 g of iron nitrate (Fe(NO$_3$)$_3$·9H$_2$O) and 144 g of nickel nitrate (Ni(NO$_3$)$_2$·6H$_2$O) were dissolved in 500 ml of pure water, and 100 g of sodium hydroxide were dissolved in 500 ml of pure water. Both solutions were added dropwise to 2$\ell$ of pure water at ordinary temperature so as to maintain a pH of 7-8. After adding, the mixture was stirred for 1 h. The precipitates produced were filtered and washed. Subsequently, 100 ml of aqueous solution containing 1.77 g of sodium carbonate (Ni$_2$CO$_3$· 10H$_2$O) and 0.16 g of calcium nitrate (Ca(NO$_3$)$_2$·4H$_2$O) were added to the gelatinous material, and stirred for 1 h. The gelatinous material was dried in the atmosphere at 120°C for 24 h, and then calcined in the atmosphere at 800°C for 4 h to obtain a catalyst having a ratio by weight of 51.4 : 48.1 : 0.5 : 0.05 as Fe$_2$O$_3$ :NiO : Na$_2$O : CaO.

Example 42

Using 1.62 g of calcium nitrate, a catalyst was prepared with the some method as in Examples 39 to 41. The catalyst obtained had a ratio by weight of 51.2 : 47.8 : 0.5 : 0.5 as Fe$_2$O$_3$ : NiO : Na$_2$O : CaO.

Production of Phenol

Each catalyst was crushed and sieved to a prescribed mesh size, and the amount described in Table 8 was placed in a quartz tube having an inner diameter of 20 mm. Benzoic acid, water vapor, air and nitrogen gas were supplied at the rate described in Table 8, and allowed to react at the temperature described in Table 8.

The experimental results are summarized in Table 8.

Table 8

| Catalyst | Example 39 | Example 40 | Example 41 | Example 42 |
|---|---|---|---|---|
| Composition (wt. ratio) | $Fe_2O_3$-NiO-$Na_2O$-CaO (51.4:48.1:0.5:0.05) | $Fe_2O_3$-NiO-$Na_2O$-CaO (51.4:48.1:0.5:0.05) | $Fe_2O_3$-NiO-$Na_2O$-CaO (51.4:48.1:0.5:0.05) | $Fe_2O_3$-NiO-$Na_2O$-CaO (51.2:47.8:0.5:0.5) |
| Calcining Temperature (°C) | 800 | 800 | 800 | 800 |
| Used Amount (ml) | 3.85 | 3.85 | 3.85 | 3.85 |
| Reacting Conditions | | | | |
| Reaction Temperature (°C) | 400 | 400 | 400 | 400 |
| Supply Gas Concentration (%) | | | | |
| Benzoic Acid | 6.8 | 7.8 | 7.9 | 7.3 |
| Water Vapor | 67.6 | 69.8 | 71.4 | 65.7 |
| Oxygen Gas | 5.1 | 4.5 | 4.1 | 5.4 |
| Nitrogen Gas | 20.5 | 18.0 | 16.5 | 21.6 |
| Space Velocity ($h^{-1}$) | 7350 | 8770 | 9970 | 7630 |
| Reaction Results | | | | |
| Conversion of Benzoic Acid (%) | 93.7 | 78.3 | 68.7 | 74.8 |
| Selectivity (%) | | | | |
| Phenol | 93.1 | 92.0 | 91.4 | 87.6 |
| Benzene | 5.9 | 6.8 | 6.5 | 11.5 |
| CO, $CO_2$ | tr | tr | 0.5 | 2.0 |
| Yield of Phenol (%) | 93.7x0.931 | 78.3x0.920 | 68.7x0.914 | 74.8x0.876 |
| Space Time Yield of Phenol ($g/\ell \cdot h$) | 1829 | 2053 | 2086 | 1531 |

EP 0 452 695 B1

**Claims**

1.  A process for producing a catalyst consisting essentially of iron oxide and nickel oxide which comprises calcining a mixture of iron oxide or a precursor thereof with nickel oxide or a precursor thereof at a temperature of 600 to 900°C.

2.  The process of claim 1, wherein the ratio by weight of nickel oxide/iron oxide of the catalyst is 0.1 to 10.

3.  The process of claim 1, wherein the ratio by weight of nickel oxide/iron oxide of the catalyst is 0.5 to 2.

4.  The process of claim 1, wherein an alkali metal compound is added to the pre- or post-calcined mixture

5.  The process of claim 4, wherein said alkali metal compound is selected from oxides, hydroxides, carbonates and nitrates.

6.  The process of claim 5, wherein said alkali metal compound is an oxide.

7.  The process of claim 6, wherein said oxide is sodium oxide or potassium oxide.

8.  The process of claim 4, wherein the content of the alkali metal compound converted to the oxide thereof in the catalyst is 0.05 to 30 wt. %.

9.  The process of claim 8, wherein the ratio by weight of nickel oxide/iron oxide in the catalyst is 0.1 to 10.

10. The process of claim 1, wherein an alkaline earth metal compound is added to the pre- or post-calcined mixture.

11. The process of claim 10, wherein said alkaline earth metal compound is selected from oxides, hydroxides, carbonates and nitrates.

12. The process of claim 10, wherein said alkaline earth metal compound is an oxide.

13. The process of claim 12, wherein said oxide is calcium oxide.

14. The process of claim 10, wherein the content of the alkaline earth metal compound converted to the oxide thereof in the catalyst is 0.05 to 30 wt. %.

15. The process of claim 14, wherein the ratio by weight of nickel oxide/iron oxide in the catalyst is 0.1 to 10.

16. The process of claim 1, wherein the catalyst contains an alkali metal compound and an alkaline earth metal compound.

17. The process of claim 15, wherein both of said alkali metal compound and alkaline earth metal compound are oxides.

18. The process of claim 17, wherein said alkali metal compound is sodium oxide and said alkaline earth metal compound is calcium oxide.

19. The process of claim 18, wherein the content of the alkali metal compound converted to the oxide thereof in the catalyst is 0.01 to 20 wt. % and the content of the alkaline earth metal compound converted to the oxide thereof in the catalyst is 0.01 to 20 wt. %.

20. The use of a catalyst consisting essentially of iron oxide and nickel oxide prepared by the process according to any of claims 1 to 19 in a process for producing phenol from benzoic acid through vapor phase oxidation.

21. The use of claim 20, wherein the catalyst is supported on a carrier.

23

**Patentansprüche**

1. Verfahren zum Herstellen eines Katalysators, bestehend im wesentlichen aus Eisenoxid und Nickeloxid, welches das Calcinieren eines Gemisches aus Eisenoxid oder einem Vorläufer davon mit Nickeloxid oder einem Vorläufer davon bei einer Temperatur von 600 bis 900°C umfaßt.

2. Verfahren nach Anspruch 1, wobei das Gewichtsverhältnis von Nickeloxid/Eisenoxid des Katalysators 0,1 bis 10 beträgt.

3. Verfahren nach Anspruch 1, wobei das Gewichtsverhältnis von Nickeloxid/Eisenoxid des Katalysators 0,5 bis 2 beträgt.

4. Verfahren nach Anspruch 1, wobei eine Alkalimetallverbindung dem vor- oder nach-calcinierten Gemisch zugefügt wird.

5. Verfahren nach Anspruch 4, wobei die Alkalimetallverbindung ausgewählt ist aus Oxiden, Hydroxiden, Carbonaten und Nitraten.

6. Verfahren nach Anspruch 5, wobei die Alkalimetallverbindung ein Oxid ist.

7. Verfahren nach Anspruch 6, wobei das Oxid Natriumoxid oder Kaliumoxid ist.

8. Verfahren nach Anspruch 4, wobei der Gehalt der Alkalimetallverbindung, umgewandelt in das Oxid davon, in dem Katalysator 0,05 bis 30 Gew.-% beträgt.

9. Verfahren nach Anspruch 8, wobei das Gewichtsverhältnis von Nickeloxid/Eisenoxid in dem Katalysator 0,1 bis 10 beträgt.

10. Verfahren nach Anspruch 1, wobei eine Erdalkalimetallverbindung dem vor- oder nach-calcinierten Gemisch zugefügt wird.

11. Verfahren nach Anspruch 10, wobei die Erdalkalimetallverbindung ausgewählt ist aus Oxiden, Hydroxiden, Carbonaten und Nitraten.

12. Verfahren nach Anspruch 10, wobei die Erdalkalimetallverbindung ein Oxid ist.

13. Verfahren nach Anspruch 12, wobei das Oxid Calciumoxid ist.

14. Verfahren nach Anspruch 10, wobei der Gehalt der Erdalkalimetallverbindung, umgewandelt in das Oxid davon, in dem Katalysator 0,05 bis 30 Gew.-% ist.

15. Verfahren nach Anspruch 14, wobei das Gewichtsverhältnis von Nickeloxid/Eisenoxid in dem Katalysator 0,1 bis 10 beträgt.

16. Verfahren nach Anspruch 1, wobei der Katalysator eine Alkalimetallverbindung und eine Erdalkalimetallverbindung enthält.

17. Verfahren nach Anspruch 15, wobei sowohl die Alkalimetallverbindung als auch die Erdalkalimetallverbindung ein Oxid ist.

18. Verfahren nach Anspruch 17, wobei die Alkalimetallverbindung Natriumoxid und die Erdalkalimetallverbindung Calciumoxid ist.

19. Verfahren nach Anspruch 18, wobei der Gehalt der Alkalimetallverbindung, umgewandelt in das Oxid davon, in dem Katalysator 0,01 bis 20 Gew.-% und der Gehalt der Erdalkalimetallverbindung, umgewandelt in das Oxid davon, in dem Katalysator 0,01 bis 20 Gew.-% beträgt.

20. Verwendung eines Katalysators, bestehend im wesentlichen aus Eisenoxid und Nickeloxid, hergestellt gemäß dem Verfahren nach einem der Ansprüche 1 bis 19, in einem Verfahren zur Herstellung von Phenol aus Benzoesäure durch Dampfphasenoxidation.

**21.** Verwendung nach Anspruch 20, wobei der Katalysator von einem Träger getragen wird.


**Revendications**

**1.** Procédé pour produire un catalyseur essentiellement constitué d'oxyde de fer et d'oxyde de nickel qui comprend la calcination d'un mélange d'oxyde de fer ou d'un précurseur de celui-ci avec de l'oxyde de nickel ou un précurseur de celui-ci, à une température de 600 à 900°C.

**2.** Procédé selon la revendication 1, dans lequel le rapport pondéral de l'oxyde de nickel à l'oxyde de fer du catalyseur est de 0,1 à 10.

**3.** Procédé selon la revendication 1, dans lequel le rapport pondéral de l'oxyde de nickel à l'oxyde de fer du catalyseur est de 0,5 à 2.

**4.** Procédé selon la revendication 1, dans lequel un composé de métal alcalin est ajouté au mélange pré- ou post-calciné.

**5.** Procédé selon la revendication 4, dans lequel ledit composé de métal alcalin est sélectionné parmi des oxydes, des hydroxydes, des carbonates, et des nitrates.

**6.** Procédé selon la revendication 5, dans lequel ledit composé de métal alcalin est un oxyde.

**7.** Procédé selon la revendication 6, dans lequel ledit oxyde est l'oxyde de sodium ou l'oxyde de potassium.

**8.** Procédé selon la revendication 4, dans lequel la teneur en composé de métal alcalin converti en son oxyde dans le catalyseur, est de 0,05 à 30 % en poids.

**9.** Procédé selon la revendication 8, dans lequel le rapport pondéral de l'oxyde de nickel à l'oxyde de fer dans le catalyseur est de 0,1 à 10.

**10.** Procédé selon la revendication 1, dans lequel un composé de métal alcalino-terreux est ajouté au mélange pré- ou post-calciné.

**11.** Procédé selon la revendication 10, dans lequel ledit composé de métal alcalino-terreux est sélectionné parmi des oxydes, des hydroxydes, des carbonates et des nitrates.

**12.** Procédé selon la revendication 10, dans lequel ledit composé de métal alcalino-terreux est un oxyde.

**13.** Procédé selon la revendication 12, dans lequel ledit oxyde est l'oxyde de calcium.

**14.** Procédé selon la revendication 10, dans lequel la teneur en composé de métal alcalino-terreux converti en son oxyde dans le catalyseur, est de 0,05 à 30% en poids.

**15.** Procédé selon la revendication 14, dans lequel le rapport pondéral de l'oxyde de nickel à l'oxyde de fer dans le catalyseur est de 0,1 à 10.

**16.** Procédé selon la revendication 1, dans lequel le catalyseur contient un composé de métal alcalin et un composé de métal alcalino-terreux.

**17.** Procédé selon la revendication 15, dans lequel le composé de métal alcalin et le composé de métal alcalino-terreux sont tous deux des oxydes.

**18.** Procédé selon la revendication 17, dans lequel ledit composé de métal alcalin est l'oxyde de sodium et ledit composé de métal alcalino-terreux est l'oxyde de calcium.

**19.** Procédé selon la revendication 18, dans lequel la teneur en composé de métal alcalin converti en son oxyde dans le catalyseur, est de 0,01 à 20% en poids et la teneur en composé de métal alcalino-terreux, converti en son oxyde dans le catalyseur, est de 0,01 à 20% en poids.

**20.** Utilisation d'un catalyseur essentiellement constitué d'oxyde de fer et d'oxyde de nickel préparé par le procédé selon l'une quelconque des revendications 1 à 19, dans un procédé de production de phénol à partir d'acide benzoïque par oxydation en phase vapeur.

21. Utilisation selon la revendication 20, dans laquelle le catalyseur est porté par un véhicule.